(19) **Europäisches Patentamt / European Patent Office / Office européen des brevets**

(11) **EP 2 772 549 B1**

(12) # EUROPEAN PATENT SPECIFICATION

(45) Date of publication and mention of the grant of the patent:
**31.07.2019 Bulletin 2019/31**

(51) Int Cl.:
*C12Q 1/6869* (2018.01)  *C12Q 1/6809* (2018.01)
*G16B 30/00* (2019.01)

(21) Application number: **11878559.1**

(22) Date of filing: **31.12.2011**

(86) International application number:
**PCT/CN2011/002244**

(87) International publication number:
**WO 2013/097062 (04.07.2013 Gazette 2013/27)**

(54) **METHOD FOR DETECTING GENETIC VARIATION**

VERFAHREN ZUM NACHWEIS VON GENETISCHEN VARIATIONEN

PROCÉDÉ DE DÉTECTION D'UNE VARIATION GÉNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.09.2014 Bulletin 2014/36**

(73) Proprietor: **BGI Diagnosis Co., Ltd.**
**Guangdong 518083 (CN)**

(72) Inventors:
 • **CHEN, Shengpei**
  **Shenzhen**
  **Guangdong 518083 (CN)**
 • **ZHANG, Chunlei**
  **Shenzhen**
  **Guangdong 518083 (CN)**
 • **CHEN, Fang**
  **Shenzhen**
  **Guangdong 518083 (CN)**
 • **XIE, Weiwei**
  **Shenzhen**
  **Guangdong 518083 (CN)**
 • **PAN, Xiaoyu**
  **Shenzhen**
  **Guangdong 518083 (CN)**
 • **WANG, Jian**
  **Shenzhen**
  **Guangdong 518083 (CN)**
 • **WANG, Jun**
  **Shenzhen**
  **Guangdong 518083 (CN)**

 • **YANG, Huanming**
  **Shenzhen**
  **Guangdong 518083 (CN)**
 • **ZHANG, Xiuqing**
  **Shenzhen**
  **Guangdong 518083 (CN)**

(74) Representative: **HGF Limited**
**8th Floor**
**140 London Wall**
**London EC2Y 5DN (GB)**

(56) References cited:
**WO-A1-2010/042716   WO-A1-2010/054284**
**WO-A1-2010/057132   WO-A1-2010/075459**
**WO-A2-2010/001419   CN-A- 101 555 528**

 • **S. YOON ET AL: "Sensitive and accurate detection of copy number variants using read depth of coverage", GENOME RESEARCH, vol. 19, no. 9, 5 August 2009 (2009-08-05) , pages 1586-1592, XP055167321, ISSN: 1088-9051, DOI: 10.1101/gr.092981.109**
 • **ERIC Z. CHEN ET AL: "Noninvasive Prenatal Diagnosis of Fetal Trisomy 18 and Trisomy 13 by Maternal Plasma DNA Sequencing", PLOS ONE, vol. 6, no. 7, 6 July 2011 (2011-07-06), page e21791, XP055024137, DOI: 10.1371/journal.pone.0021791**

**(Cont. next page)**

- Anonymous: "Resampling (statistics) - Wikipedia, the free encyclopedia", , 29 December 2011 (2011-12-29), XP055168025, Retrieved from the Internet: URL:http://en.wikipedia.org/w/index.php?title=Resampling_(statistics)&oldid=46831285 8 [retrieved on 2015-02-06]

- Daisuke Komura ET AL: "Noise reduction from genotyping microarrays using probe level information", In silico biology, 1 January 2006 (2006-01-01), pages 79-92, XP055168063, Netherlands Retrieved from the Internet: URL:http://www.ncbi.nlm.nih.gov/pubmed/167 89916 [retrieved on 2015-02-06]

## Description

## Technical Field

The present invention relates to the field of genetic variation detection, and in particular, to the detection of a copy number variation, e.g., microdeletion/microduplication and aneuploidy.

## Background Art

A copy number variation (CNV) refers to a submicroscopic mutation of a DNA fragment in a range from kb to Mb, which is marked by the increase or decrease in the copy number. Research on the relationship between copy number variation and disease has a long history. For some germline mutation copy number variations (i.e. copy number variations generated due to variations of a fetus itself, which are absent in both of the parents), it is believed that the larger a fragment is, the easier a congenital anomaly occurs, and for example, chromosomal aneuploidy diseases (e.g. T21, T18 etc.) and chromosomal microdeletion/microduplication syndromes are all recognized germline mutation copy number variation related diseases.

Human chromosomal microdeletion/microduplication syndromes are a disease type of complex and changeable phenotypes caused by the occurrence of micro-fragment deletions or duplications, i.e., copy number variations in DNA fragments, on human chromosomes with a relatively high incidence in perinatal infants and neonatal infants, and can lead to serious diseases and abnormalities, e.g., congenital heart disease or heart malformation, serious growth retardation, appearance or limb deformity, etc. In addition, the microdeletion syndromes are also one of the main reasons causing mental retardation besides Down's syndrome and fragile X syndrome (Knight SJL (ed): Genetics of Mental Retardation. Monogr Hum Genet. Basel, Karger, 2010, vol 18, pp 101 - 113 (DOI: 10.1159/000287600)). In recent years, the congenital heart disease topping the list in the statistics for the incidence of birth defects and the mental retardation, cerebral palsy and congenital deafness which are top-ranked in outpatient clinics for genetic counseling and diagnosis are all related to the microdeletion syndromes. Common microdeletion syndromes include 22q11 microdeletion syndrome, cri du chat syndrome, Angelman syndrome, AZF deletion, etc.

Although the incidence of each microdeletion syndrome is very low, wherein the incidences of the relatively common 22q11 microdeletion syndrome, cri du chat syndrome, Angelman syndrome, Miller-Dieker syndrome, etc. are 1 : 4,000 (live births), 1 : 50,000, 1 : 10,000 and 1 : 12,000 respectively. Due to the limitation by clinical detection techniques, a large number of patients with microdeletion syndromes cannot be detected in prenatal screening and prenatal diagnosis. And even when a reason is looked for retrospectively after the occurrence of typical clinical characterizations months or even years after the birth of an infant, the cause of the disease also cannot be diagnosed due to the limitation by the detection techniques (https://decipher.sanger.ac.uk/syndromes) . Because a radical cure cannot be effected for some types of microdeletion syndromes with the death within months or years after the birth, a heavy mental and economic burden is brought to the society and families. According to incomplete statistics, patients with "happy puppet syndrome" (Angelman syndrome) worldwide have reached 15 thousand, and the numbers of patients with the other types of chromosomal microdeletion syndromes are also increasing year by year. Thus, the pre-pregnancy detection of chromosomal microdeletions/microduplications performed on clinically suspected patients and parents with a related abnormal pregnancy history is conducive to providing genetic counseling and providing a basis for clinical decision making. The early prenatal diagnosis during pregnancy can effectively prevent the birth of an infant patient or provide a basis for providing a treatment approach for an infant patient after birth (Bretelle F, et al. Prenatal and postnatal diagnosis of 22q11.2 deletion syndrome. Eur J Med Genet. 2010 Nov-Dec;53(6):367-70).

However, this type of diseases cannot be detected by routine clinical methods such as the chromosome karyotyping method and the like (with a resolution of above 10 M) because of the micro level of variation in chromosome (Malcolm S.Microdeletion and microduplication syndromes. Prenat Diagn. 1996 Dec;16(13): 1213-9). Currently, for the prenatal diagnosis of the microdeletion/microduplication syndromes, methods of invasive fetal amniotic fluid or other tissues are mainly adopted to perform the molecular diagnosis. Currently, invasive molecular diagnostic methods mainly include high-resolution chromosome karyotyping, FISH (fluorescence in situ hybridization), Array CGH (comparative genomic hybridization), MLPA (multiplex ligation-dependent probe amplification technique), PCR and the like. Among them, the FISH examination is used as a gold standard for genetic diagnosis which can effectively detect most of deletions of chromosomal fragments. However, because invasive sampling needs a certain surgery or cell culture, from the point of time efficiency and resource consumption, same is suitable for acting as a diagnostic indicator, but not a method for universal clinical screening.

In terms of non-invasive screening methods for the microdeletion/microduplication syndromes, there are also some attempts. For example, in a study on the non-invasive detection of the microdeletion syndromes in a fetus published in November 2011, the researchers performed high depth sequencing on plasma of the mother during pregnancy, generated about 243 million short reads, and detected a microdeletion of around 4 Mb from 12p 11.22 to 12p 12.1 in the fetus (David Peters, et al. Noninvasive Prenatal Diagnosis of a Fetal Microdeletion Syndrome .N Engl J Med 2011; 365:1847-1848). However, the generation of such a large amount of data is not suitable for clinical use in terms of either resource consumption or time efficiency.

It can be known from the combination of the above-mentioned contents that currently, among the prenatal examination methods for chromosomal microdeletion/microduplication syndromes, there is still no feasible universal screening method. A new credible screening method for copy number variation in a fetus is needed in this field, so as to identity known sites and to explore unknown sites discoverably.

## Summary of the Invention

With the continuous development of the high-throughput sequencing technique and the continuous reduction in the sequencing cost, studies on sequencing techniques in prenatal screening make analysis of screening of chromosomal copy number variation and aneuploidy and other genetic variations, and in particular, fetal aneuploidy chromosomal variation, by high-throughput sequencing, be more and more widely applied. In order to detect genetic variation, the present invention designs a genetic variation screening method based on the high-throughput sequencing technique which can use the detection of copy number variation and aneuploidy and other genetic variations and has the features of high throughput, high specificity and accurate location.

Accordingly, the present invention provides a computer-implemented method of detecting genetic variation as set forth in any of claims 1 to 15.

Described herein is the acquiring of a test sample and extracting DNA, performing high-throughput sequencing and analyzing the obtained data to obtain a detection result.

Also described herein is a method for detecting genetic variation, comprising the following steps:

1) acquiring reads from a test sample, wherein the fragment of said reads, for example, can be 25-100 nt in length, and the fragment number of said reads can be at least 1 million;
2) aligning said reads with a reference genome sequence;
3) dividing said reference genome sequence into windows, calculating the number of reads which are aligned to each window, and acquiring the statistic for each window on the basis of the number of said reads;
4) and for a fragment of the reference genome sequence, on the basis of the change in the statistics of all the windows thereon in the fragment of the reference genome sequence, acquiring positions where a significant change occurs in statistics of the windows on both sides, these positions being positions where genetic variation sites of the test sample are on the reference genome sequence.

Said genetic variation site in accordance with the method of the invention is the median point between an inflection point where said statistic turns from ascending to descending and the next same inflection point, and there is at least 50, at least 70, at least 100, preferably 100 window lengths between two genetic variation sites; and the above-mentioned site, inflection point and median point refer to a chromosomal position corresponding to a window corresponding to the statistic, and can be represented by the starting point, the midpoint, the end point and any other position of the window.

As described herein, there is the step 5) of screening the genetic variation sites to obtain post-screening genetic variation sites.

For example, the above-mentioned step 5) is for each genetic variation site comprises performing statistics on the difference between two numerical groups consisting of statistics of windows contained in the fragment between the genetic variation site and its preceding genetic variation site and in the fragment between the genetic variation site and its subsequent variation site, and removing the genetic variation site whose significance value of difference is maximum and greater than a preset threshold; and repeating the above-mentioned process, until the significance values of difference of the genetic variation sites are all smaller than the preset threshold, wherein said significance of difference, e.g., can be performed by the run test, removing the genetic variation site whose significance value in the run test is maximum and greater than the preset threshold; and repeating the above-mentioned process, until the significance values of the genetic variation sites in the run test are all smaller than the preset threshold.

Also as described, the preset threshold used in the above-mentioned step 5) is obtained by the following steps:

a) acquiring the genetic variation sites according to the method of the present invention by substituting the test sample with a control sample;
b) for each genetic variation site, performing statistics on the difference between two numerical groups consisting of statistics of windows contained in the fragment between the genetic variation site and its preceding genetic variation site and in the fragment between the genetic variation site and its subsequent variation site, and removing the genetic variation site which is the least significant; and

c) repeating the above-mentioned step b), until the number of remaining candidate breakpoints is equal to the expected value $N_c$, wherein $N_c = L_c/T$, $L_c$ is the length of the genome sequence, the theoretical ultimate precision $T$ is the fragment size which can be detected theoretically, the theoretical ultimate precision $T = W + S*N$ when the average of the window sizes is W, the sliding length of the windows is S and the number of each window group in the run test is N, and among significance values of all the remaining candidate breakpoints, the minimum is the significance threshold.

As described herein, there is also a method for detecting genetic variation, comprising the following steps:

1) acquiring the genetic variation sites on a fragment of the reference genome sequence according to the method of the present invention;
2) and a step of performing a confidence-based selection on fragments between said genetic variation sites.

The step of confidence-based selection in the above-mentioned step 2) may comprise:

i) calculating the distribution probability of the statistics through the distribution pattern of the statistics for the windows, and setting a threshold;
ii) and comparing the average of the statistics of windows in the fragment between post-screening genetic variation sites with said threshold, and determining whether the fragment between the genetic sites is anomalous on the basis of the comparison result.

In another way, the step of confidence-based selection in the above-mentioned step 2) comprises:

i) calculating the distribution probability of the statistics through the distribution pattern of the statistics for the windows, and setting a first threshold and a second threshold;
ii) and comparing the average of the statistics of windows in the fragment between post-screening genetic variation sites with said first threshold and second threshold,

wherein, if the statistics for windows in the fragment are smaller than the first threshold, the fragment is a fragment deletion, and if same are greater than the second threshold, the fragment is a fragment duplication,
wherein said first threshold is a value of the statistic where the cumulative probability of the occurrence of the statistic is less than or equal to 0.1, preferably less than or equal to 0.01, most preferably 0.05, and/or said second threshold can be a value of the statistic where the cumulative probability of the occurrence of the statistic is greater than or equal to 0.9, preferably greater than or equal to 0.99, most preferably 0.95.

Further described herein is a computer-readable medium, carrying a series of executable codes, which can execute the method of genetic detection of the invention and as described herein.

Also described is a method for detecting fetal genetic variation, comprising the following steps:

acquiring a maternal sample containing fetal nucleic acid;
sequencing said maternal sample;
and a step of detecting the genetic variation using the method as described herein. Said maternal sample may be maternal peripheral blood.

The superiority of the present invention, compared with the current methods for detecting genetic variation, mainly includes the following points:

(1) Being clinically feasible: we use only around 5 M of sequencing data, and can detect around 5 Mb of CNV fragments, while a reported method used that of nearly 243 M, therefore, our method reduces the cost and time consumption of data generation greatly.
(2) Being extensible: we can increase the precision by expanding the number of control groups besides by increasing the amount of sequencing, so as to reduce the pressure on the starting amount of DNA.
(3) Being more stable and more comprehensive: there is no detail of the operation itself pointed out clearly in reported articles, while the present invention designs the correction of data groups, the preference for fragmentation conditions and various other aspects.

## Description of the Drawings

Figure 1 is a brief flowchart on the genetic variation analysis of chromosomes in an example of the present invention.
Figure 2A is a digital chromosomal karyogram of S67.
Figure 2B is a digital chromosomal karyogram of S10.
Figure 2C is a digital chromosomal karyogram of S14.
Figure 2D is a digital chromosomal karyogram of S18.
Figure 2E is a digital chromosomal karyogram of S49.
Figure 2F is a digital chromosomal karyogram of S55.
Figure 2G is a digital chromosomal karyogram of S82.
Figure 2H is a digital chromosomal karyogram of S103.

Description of the Tables

Table 1 is a list of CNV results of all samples in the implementation case.
Table 2 shows aCGH and karyotyping detection results of all samples in the implementation case.
Table 3. shows the test results in the present implementation case and the results of standard karyotyping detection.

**Embodiments**

The type of the nucleic acid is not particularly limited, which can be deoxyribonucleic acid (DNA), and can also be ribonucleic acid (RNA), preferably DNA. It would be understood by those skilled in the art that for RNA, same can be converted by conventional means into DNA with a corresponding sequence to perform subsequent detection and analysis. In addition, the property of the test sample is not particularly limited either. A genomic DNA sample can be adopted, and a part of genomic DNA can also be adopted as the test sample. The source of the test sample is not particularly limited. A sample from a pregnant woman can be adopted as the test sample, from which a nucleic acid sample containing fetal genetic information can thereby be extracted, and then the fetal genetic information and physiological state can be detected and analyzed. Examples of a sample from a pregnant woman which can be used include, but are not limited to, peripheral blood of the pregnant woman, urine of the pregnant woman, cervical fetal exfoliated trophoblastic cells of the pregnant woman, cervical mucus of the pregnant woman, and fetal nucleated red blood cells. The inventors found that through the extraction of the nucleic acid sample from the above-mentioned sample of a pregnant woman, the genetic variation in the genome of a fetus can be analyzed effectively to realize the non-invasive prenatal diagnosis or detection on the fetus. Advantageously, non-invasive detection of fetal genetic variation can be performed, for example, said sample is peripheral blood of a pregnant woman, moreover, invasive detection is possible, for example, said sample can be from fetal cord blood; said tissue can be placental tissue or chorionic tissue; and said cells can be uncultured or cultured amniotic fluid cells and villus progenitor cells. A subject to be tested and a normal subject are of the same species. Furthermore, the variation detection of the present invention is not necessarily used for diagnosis of diseases or related purposes, because with the presence of polymorphism, the presence of some variations relative to a reference genome does not represent the risk of suffering from a disease or the state of health. Thus, the variation detection of the present invention can be simply for use in scientific research on genetic polymorphism.

In the present invention, a control sample is against the test sample. For example, in a method related to the detection of a disease, the control sample refers to a normal sample. For example, in an embodiment of the present invention, the test sample is maternal peripheral blood, and the corresponding control sample is peripheral blood of a normal mother conceiving a normal fetus.

As described herein, the method and apparatus for extracting the nucleic acid sample from the test sample is not particularly limited either, and commercialized nucleic acid extraction kits can be adopted to perform same.

In the method of the present invention, said windows have the same number of reference unique reads. The reference unique reads refer to a chromosomal fragment with a unique sequence, this fragment can be definitely located at a single chromosomal position, and the chromosomal reference unique reads can be constructed based on a disclosed chromosomal reference genome sequence, e.g., hg18 or hg19. A process for acquiring the reference unique reads generally include the steps of cutting the reference genome into reads of any fixed length, aligning these reads back to the reference genome, and selecting the reads which are aligned to the reference genome uniquely as the reference unique reads. Said fixed length depends on the lengths of sequences in the sequencing result by a sequencer, referring to the average length for detail. The lengths in the sequencing results obtained by different sequencers are different, and specifically for each run of sequencing, the lengths in the sequencing results may also be different, and there are certain subjective and experience factors existing in the selection of the length.

In an example of the present invention, the length of the reference unique reads is selected according to the actual lengths of sequences in the sequencing result, e.g. 25-100 bp, and for the illumina/Solexa system, e.g. optionally 50 bp, and then the number of reference unique reads contained in each window is controlled at 800,000-900,000. In the method of the present invention, said windows can have an overlap or have no overlap therebetween. In an example of the present invention, the distance between adjacent windows is 1-100 kb, preferably 5-20 kb, more preferably 10 kb. This distance can be adjusted according to the DNA abundance in the fetal sample. The principle of the adjustment is that each window corresponds to one statistic and one chromosomal position, which also means that the distance between windows determines the precision of detection. The higher the precision is, the higher the maternal derived background is, and the more difficult the discrimination of sources of genetic variations is.

In the method of the present invention, said statistic may be the number of reads itself, but preferably is a statistic after error correction (e.g. GC correction) and/or data standardization, the purpose of which is that the statistic meets a common distribution in the statistics, e.g. the normal or standard normal distribution. The subsequent statistical analysis of the statistics can thus be facilitated. In an example of the present invention, the standardization processing against the average number of reads of all the windows is performed. In an example of the present invention, the standardization includes a process for evaluating the Z value hereinafter. In an embodiment, said statistic approximately fits normal distribution obtained by the standardization processing on the number of reads which are aligned to a window. In an embodiment, said standardization is based on the average number of reads which are aligned to all the windows. In an embodiment, said statistic is an approximate standard normal distribution statistic.

In the present invention, the reads refer to sequence fragments outputted by a sequencer, preferably about 25-100 nt.

DNA molecules can be acquired using the salting-out method, the column chromatography method, the magnetic bead method, the SDS method and other routine DNA extraction methods, preferably using the magnetic bead method. The so-called magnetic bead method refers to for bare DNA molecules obtained after the blood, tissues or cells undergo the action of a cell lysis solution and proteinase K, using specific magnetic beads to perform reversible affinity adsorption on the DNA molecules, and after proteins, lipids and other impurities are removed by washing with a rinsing liquid, eluting the DNA molecules from the magnetic beads with a purification liquid. The magnetic beads are well known in the art, and are commercially available, e.g. from Tiangen.

Generally, the direct sequencing of the DNA molecules obtained from the samples and subsequent steps is undertaken, and extracted DNA can be used for subsequent steps without being processed. In some preferred examples, fragments with electrophoretic main bands concentrated in the size of 50-700 bp, preferably 100-500 bp, more preferably 150-300 bp, particularly about 200 bp, may only be studied. In some more preferred examples, the DNA molecules can be broken into fragments with electrophoretic main bands concentrated in a certain size, e.g., 50-700 bp, preferably 100-500 bp, more preferably 150-300 bp, particularly near 200 bp, and then the subsequent steps are performed. The treatment of randomly breaking said DNA molecules can use enzyme digestion, atomization, ultrasound or the Hydro-Shear method. Preferably, the ultrasound method is used, for example, the S-series of the Covaris Corporation (based on the AFA technique, wherein when the sound energy/mechanical energy released by a sensor passes through a DNA sample, gas is dissolved to form bubbles; after removing the energy, the bubbles burst and the ability to fracture DNA molecules is generated; through setting of a certain energy intensity and time interval and other conditions, the DNA molecules can be broken into sizes within a certain range; for example, for a specific principle and method, see the instructions for the S-series from the Covaris Corporation).

As described herein, said breakpoint or candidate breakpoint is a potential or existing genetic variation site, and by convention, the site is expressed as the position on the reference genome. The two concepts, genetic variation site and breakpoint, are interchangeable in a particular case, and just different in expression, and may both be used to represent the position coordinate of a potential or definitely existing genetic variation on the reference genome in various stages.

The method of sequencing can be adopted to acquire the reads from the test sample, and said sequencing can be performed through any sequencing method, which includes, but is not limited to, the dideoxy chain-termination method; preferably a high-throughput sequencing method, which includes, but is not limited to, second-generation sequencing techniques or single molecule sequencing techniques (Rusk, Nicole (2009-04-01). Cheap Third-Generation Sequencing. Nature Methods 6 (4): 2446 (4) .

Platforms for said second-generation sequencing (Metzker ML. Sequencing technologies-the next generation. Nat Rev Genet. 2010 Jan;11(1):31-46) include, but are not limited to, Illumina-Solexa (GATM, HiSeq2000TM, etc.), ABI-Solid and Roche-454 (pyrosequencing) sequencing platform; and platforms (techniques) for the single molecule sequencing include, but are not limited to, True Single Molecule DNA sequencing from the Helicos Corporation, single molecule real-time sequencing (SMRTTM) from the Pacific Biosciences Corporation, and nanopore sequencing technique from the Oxford Nanopore Technologies Corporation, etc.

The type of sequencing can be single-end sequencing and pair-end sequencing, and the sequencing length can be 50 bp, 90 bp or 100 bp. In an embodiment of the present invention, said sequencing platform is Illumina/Solexa, the type of sequencing is pair-end sequencing, and 100 bp sized DNA molecule sequence with the pair-end positional relationship is obtained.

As described, the sequencing depth of sequencing can be determined according to the size of fetal chromosomal variation fragment to be detected, and the higher the sequencing depth is, the higher the detection sensitivity is, i.e., the smaller the detectable deletion and duplication fragment is. The sequencing depth can be 1 - 30 ×, i.e., the total amount of data is 1-30 times the length of the human genome, for example, in an embodiment of the present invention, the sequencing depth is 0.1 ×, i.e., 2 times (2.5 × 108 bp).

When the DNA molecules to be tested are from a plurality of test samples, a different tag sequence can be added to each sample, so as to discriminate the samples in the sequencing process (Micah Hamady, Jeffrey J Walker, J Kirk Harris et al. Error-correcting barcoded primers forpyrosequencing hundreds of samples in multiplex. Nature Methods, 2008, March, Vol.5 No.3), thereby realize simultaneous sequencing of the plurality of samples. The tag sequences are for discriminating different sequences, but will not affect other functions of the DNA molecules to which the tag sequences added. The length of a tag sequence can be 4-12 bp.

Said human genomic reference sequence is preferably a human genomic reference sequence in the NCBI database, e.g. human genomic reference sequence build 36 in the NCBI database (hg18; NCBI Build 36).

As described herein, said alignment can be alignment with no mismatch allowed, and can also be alignment with 1 base mismatch allowed. The sequence alignment can be performed through any sequence alignment program, for example, the Short Oligonucleotide Analysis Package (SOAP) and the BWA (Burrows-Wheeler Aligner) alignment that are available to those skilled in the art, and the reads are aligned with the reference genome sequence to obtain the reads' positions on the reference genome. The sequence alignment can be performed using the default parameters provided by the program, or the parameters are selected by those skilled in the art according to the need. In an embodiment of the present invention, the alignment software used is SOAPaligner/soap2.

The algorithm of said software is a series of programs for detection of copy number variation in a fetus developed by the BGI institute in Shenzhen, which are collectively referred to as FCAPS. It can perform data correction, standardization and fragmentation on a test sample and a control set through data generated by the new-generation sequencing technique, and estimate the extent and size of copy number variations in a fetus.

In some particular examples, for step 1), acquiring reads from a test sample is after the extraction of plasma DNA from the test sample and the control sample according to the operation manual for Tiangen DP327-02 Kit, a library is constructed according to the modified Illumina/Solexa standard library construction flow. For details of the construction of a whole-genome sequencing library, see the directive rules provided by the manufacturer of the sequencer, e.g. the Illumina Corporation, for example, see Multiplexing Sample Preparation Guide (Part#1005361; Feb 2010) or Paired-End SamplePrep Guide (Part#1005063; Feb 2010) by the Illumina Corporation. In this process, adapters used for sequencing are added to both ends of the DNA molecules which are concentrated at 200 bp themselves, a different tag sequence is added to each sample, thereby data for a plurality of samples can be discriminated in data obtained by a single run of sequencing, and with the use of the second-generation sequencing method, Illumina/Solexa sequencing (other sequencing methods such as ABI/SOLiD can be used to achieve the same or similar effect), reads with a certain fragment size are obtained for each sample.

In some particular examples, for step 2) alignment: the reads in step 1) in the method of the present invention are SOAP2-aligned with the standard human genomic reference sequence in the NCBI database to obtain the positional information about the sequenced DNA sequence on the genome. For avoiding the disturbance to the CNV analysis by repeat sequences, only reads that are aligned with the human genomic reference sequence uniquely are selected for subsequent analysis.

In some particular examples, step 3), dividing into windows and acquiring statistics for the windows, comprises the following steps:

a) for the test sample and the control sample, providing windows with the length of w on the genomic reference sequence, calculating the GC content in each window and calculating the relative fragment number of reads falling into each window; and b) correcting and standardizing the relative fragment number of reads of the above-mentioned test sample against the relative fragment number of reads of the control sample.

In some particular examples, GC correction based on the control sample set is performed on the test sample: because a certain GC bias exists between/within sequencing batches, which makes a copy number deviation occur in the high GC region or in the low GC region in the genome, the corrected relative number of reads in each window obtained by the GC correction of sequencing data based on the control sample set can remove this bias and improve the precision of detection of copy number variation. The corrected relative number of reads in each window is standardized: copy number variation in a fetus is detected using plasma from the pregnant mother, and with the effect of the mother's DNA background, the variation in the fetus are relatively difficult to stand out, so it is demanded to reduce the noise of the mother's DNA background and amplify the signal of the copy number variation in the fetus through standardization. In an example, said GC correction comprises the following steps: a) acquiring reads which are aligned to each window according to the method of the present invention by substituting the test sample with a control sample, and calculating the relative number of the reads for each window; b) acquiring the functional relationship between the GC content of the reads which are aligned to each window and the relative number of the reads for said window; and c) for each window, using the GC content of reads of the test sample aligned to the window and the above-mentioned functional relationship, and by correcting the relative number of reads of the test sample for the window to obtain the corrected relative number of reads for the window.

In some particular examples, step 3), dividing into windows and acquiring statistics for the windows, comprises the following steps:

a) calculating the relative number of reads of the test sample and that of the control sample: for the test sample and the control sample, providing windows with the length of w on the human genomic reference sequence, calculating the number of reads, $r_{i,j}$, falling in each window in step 2) in the method of the present invention, where the subscripts i and j represent the serial number of the window and the serial number of the sample respectively, and calculating

$$R_{i,j} = \log_2 \left( \frac{r_{i,j}}{\overline{r_j}} \right)$$

the GC content, $GC_{i,j}$, of each window and calculating the relative number of reads, , where

$$\overline{r_j} = \frac{1}{n} \sum_{i=1}^{n} r_{i,j}$$

the average number of reads is ,

b) data correction and standardization:

① in a coordinate system with the GC content as the abscissa and the relative number of reads R as the ordinate, performing linear fitting on $R_{i,j}$ and $GC_{i,j}$ of the control sample to obtain the slope $a_i$ and the intercept $b_i$,

② for each window of the test sample, calculating the corrected relative number of reads

$$\overset{0}{R}_{i,j} = a_i \times GC_{i,j} + b_i,$$

③ For each window of the test sample, calculating the statistic $Z_{i,j}$:

$$Z_{i,j} = \left( R_{i,j} - \overset{0}{R}_{i,j} - mean_j \right) \Big/ SD_j, \text{ where } mean_j = \frac{1}{n} \sum_{i=1}^{n} \left( R_{i,j} - \overset{0}{R}_{i,j} \right),$$

$$SD_j = \sqrt{\frac{1}{n-1} \sum_{i=1}^{n} \left( R_{i,j} - \overset{0}{R}_{i,j} - mean_j \right)^2}.$$

In some particular examples, acquiring positions where genetic variation sites of the test sample are on the reference genome sequence in step 4) is performed through the following steps:

① initialization: for an end point of each window, if the change trend of the statistic Z is changed between the preceding window and the following window of the point, and the distance between the point and a previous point where the change trend of the statistic Z is changed between the preceding window and the following window is at least n windows (n is an integer of 10-500, preferably 50-300, e.g. 100), then the point is a candidate breakpoint, for example, the midpoint between that inflection point where the statistic Z turns from ascending to descending between the preceding window and the following window and the next same inflection point is a candidate breakpoint, or the midpoint between that inflection point where the statistic Z turns from descending to ascending between the preceding window and the following window and the next same inflection point is a candidate breakpoint bk (k = 1, 2 ,......, s; s is an integer of >0);

② optimal iteration: in order to study copy number variation or aneuploidy in a fragment of genome sequence, all sorted candidate breakpoints of the fragment of the genome sequence are recorded as $B_c = \{b_1, b_2, ..., b_s\}$, wherein two fragments, one on the left and the other on the right, exist for each candidate breakpoint $b_k$ and said fragments are a region from the previous breakpoint to the breakpoint and a region from the breakpoint to the next breakpoint;

the p value ($p_k$) obtained by subjecting $Z_{i,j}$ for all windows in these two fragments to a test (e.g., the run test which is a non-parametric test and using the uniform state of distribution after mixing of elements from two groups to evaluate the significance of difference between these two groups) is regarded as "the significance of $b_k$ as a breakpoint"; a candidate breakpoint with the maximum $p_k$ is excluded; and this step is repeated until all p values are smaller than the termination p value ($p_{final}$) for the genome sequence;

③acquisition of the termination p value: in the test process, the above-mentioned steps a) to c) ① are performed by using another control sample as the test sample, for a fragment of a genome sequence, all sorted candidate breakpoints of the fragment of the genome sequence are recorded as $B_c$ = {$b_1$, $b_2$, ..., $b_s$}, wherein two windows, one on the left and the other on the right, exist for each candidate breakpoint $b_k$; the p value ($p_k$) obtained by subjecting all $Z_{i,j}$ in these two windows to the run test is regarded as "the significance of $b_k$ as a breakpoint"; a candidate breakpoint with the maximum $p_k$ is excluded; the two windows on the left and right thereof are merged until the number of candidate breakpoints is equal to the expected value $N_c$ ($N_c$ = $L_c/T$, where $L_c$ is the length of the genome sequence c, $T$ (theoretical ultimate precision) is the fragment size which can be detected theoretically; and when the window size is W, the sliding length of the windows is S, and the number of each group in the run test is N, the theoretical ultimate precision T=W+S*N); and in the set of the candidate breakpoints, the minimum p value is the termination p value ($p_{final}$) of the genome sequence.

In some particular examples, the step of performing a confidence-based selection on fragments between said genetic variation sites is: for a fragment between genetic variation sites on the reference genome sequence, the average of $Z_{i,j}$ in the fragment is calculated and recorded as $\overline{Z}$, wherein if $\overline{Z}$ of the fragment is smaller than -1.28, then the fragment is a fragment deletion, and if same is greater than 1.28, then the fragment is a fragment duplication.

In the present invention, the run test is a non-parametric test in which, according to the uniform state of distribution of elements in two groups after mixing of the two groups, acquires the significance P value to evaluate these two groups. See http://support.sas.com/kb/33/092.html.

In the present invention, during the test with the control sample as the test sample, sequencing or experiments in practice will lead to the existence of differences in the number of fragments for sequencing to which different fragments aligned in the whole genome, so in the test process, these differences will be discriminated, and just fragments at both ends of a breakpoint have not yet reached the variation level. At the beginning of the test, these differences cannot be discriminated relatively significantly by the candidate breakpoints, so an N value is needed to be defined, which ensures that when the number of the breakpoints is the N value, the experiments can discriminate these differences relatively well, and then it can be more precise to use the threshold obtained here in testing the test sample.

For the determination of the threshold of the $\overline{Z}$ value: statistics is performed on the control sample according to steps a) and b), and then the Z value in each window meets the normal distribution, and -1.28 and 1.28 are quantiles where the cumulative probability in the normal distribution is 0.05 and 0.95, respectively. According to the need, those skilled in the art can also select the $\overline{Z}$ value as a value with a greater absolute or a smaller absolute, which correspond to a greater cumulative probability and a smaller one in the normal distribution, respectively; however, -1.28 and 1.28 are the most preferred thresholds established for the present invention by the inventors through a large number of experiments, and a threshold with a greater absolute other than the two values will increase the false negative/false positive rate in a detection result.

Non-invasive fetal CNV screening on a suitable population is conducive to providing genetic counseling and providing a basis for clinical decision making; and prenatal diagnosis can effectively prevent the birth of an infant patient. The suitable population can be all healthy pregnant women.

The following will detail the embodiments of the present invention in conjunction with examples. Those without indicated specific conditions in the examples are performed according to the routine conditions or the conditions proposed by the manufacturers. Reagents or instruments used without indicated manufacturers are all routine products available through the market. The manufacturer's article number of each reagent or kit is in the following brackets. The adapters and tag sequences used for sequencing are derived from the Multiplexing Sample Preparation Oligonutide Kit of the Illumina Corporation.

Example I. Detection of fetal large-fragment copy number variation in 1 case of maternal plasma, and detection of fetal aneuploidy variation in 9 cases of maternal plasma

1. DNA extraction:

According to the operational flow for TiangenDP327-02Kit, DNA was extracted from the above-mentioned 8 cases of plasma samples (see Table 1 for sample Nos.), a library was constructed for the extracted DNA according to the modified Illumina/Solexa standard library construction flow, adapters used for sequencing were added to both ends of DNA molecules with main bands concentrated at 200 bp, a different tag sequence was added to each sample, and then hybridization with complementary adapters on the flowcell surface was performed. A layer of single-chain primers were linked through the flowcell surface, and after turning into single chains, DNA fragments were "fixed" at one end on the chip through complementation with primer bases on the chip surface; and the other end (5' or 3') was randomly complementary to another nearby primer and was also "fixed" to form a "bridge", amplification was repeated for 30 runs, and each single molecule was amplified by about 1,000 times to form a monoclonal DNA cluster. Then through pair-end sequencing on IlluminaHiseq2000, DNA fragment sequences of about 50 bp in length were obtained.

Specifically, about 10 ng of DNA obtained from the above-mentioned plasma samples was subjected to the modified Illumina/Solexa standard library construction flow, and see the product instructions for the specific flow (the Illumina/Solexa standard library construction instructions provided by http://www.illumina.com/). The size of the DNA library was determined and the inserted fragments were determined to be about 200 bp via 2100Bioanalyzer (Agilent), and on-computer sequencing could be performed after precise quantification by QPCR.

2. Sequencing: in this example, the DNA samples obtained from the above-mentioned 10 cases of plasma were manipulated according to the instructions for ClusterStation and Hiseq2000 (PEsequencing) officially published by Illumina/Solexa to obtain the data amount of about 0.36 G from each sample to perform on-computer sequencing, and each sample was discriminated according to said tag sequences. The DNA sequences obtained by sequencing were aligned with the human genomic reference sequence build 36 in the NCBI database (hg18; NCBIBuild 36) in the manner of no mismatch allowed using the alignment software SOAP2 (obtained from soap.genomics.org.cn) to obtain locations of the DNA sequences for sequencing on said genome.

3. Data analysis

a) Calculating the relative number of reads for the test sample: the length of the reference unique reads was selected as 50 bp, the number of the reference unique reads was counted, the human genomic reference sequence was divided into windows with the same number of reference unique reads (840,000), the average of all the window sizes was 1 Mb, and the distance between adjacent windows was S = 10 kb. The actual number of reads, $r_{i,j}$, falling in each window in the above-mentioned step 2 was counted, where the subscripts $i$ and $j$ represented the serial number of the window and the serial number of the sample respectively, and the GC content of each window, $GC_{i,j}$, was calculated, and the relative

$$R_{i,j} = \log_2\left(\frac{r_{i,j}}{\overline{r}_j}\right) \qquad\qquad \overline{r}_j = \frac{1}{n}\sum_{i=1}^{n} r_{i,j}$$

number of reads, , was calculated, where the average number of reads is ;

b) Data correction and standardization:

① in a coordinate system with the GC content as the abscissa and the relative number of reads R as the ordinate, performing linear fitting on $R_{i,j}$ and $GC_{i,j}$ of the control sample to obtain the slope $a_i$ and the intercept $b_i$,

② for each window of the test sample, calculating the corrected relative number of reads

$$\overset{o}{R}_{i,j} = a_i \times GC_{i,j} + b_i,$$

③ for each window of the test sample, calculating the standardized relative number of reads $Z_{i,j}$:

$$Z_{i,j} = \left(R_{i,j} - \overset{o}{R}_{i,j} - mean_j\right)\Big/ SD_j, \text{ where } mean_j = \frac{1}{n}\sum_{i=1}^{n}\left(R_{i,j} - \overset{o}{R}_{i,j}\right),$$

$$SD_j = \sqrt{\frac{1}{n-1}\sum_{i=1}^{n}\left(R_{i,j} - \overset{o}{R}_{i,j} - mean_j\right)^2}$$

c) Window merging

① initialization: the position of the starting point of each window on the reference genome sequence was recorded as the position of the statistic Z. Then corresponding to the chromosomal position on the reference genome, the Z value had a change trend. The position corresponding to the inflection point of the Z value (i.e. a critical point where the Z value is converted from an increasing trend into a decreasing trend or turns from a decreasing trend into an increasing trend) was found. For any chromosome, positions with the distance therebetween of at least 100 windows were then selected sequentially starting from the starting point of the first window, and these positions were recorded as candidate breakpoints $b_k$ (k = 1, 2 ,......, s; s is an integer > 0);

② optimal iteration: in order to study copy number variation analysis or aneuploidy in any chromosome in the genome (in this example, only human chromosomes 1-22 were studied), all sorted candidate breakpoints of each chromosome were recorded as $B_c = \{b_1, b_2, ..., b_s\}$ , wherein two fragments, one on the left and the other on the right, exist for each candidate breakpoint $b_k$ and said fragments were a region from the previous breakpoint to the breakpoint and a region from the breakpoint to the next breakpoint; the $p$ value ($p_k$) obtained by subjecting all $Z_{i,j}$ in these two fragments to the run test was regarded as "the significance of $b_k$ as a breakpoint"; a candidate breakpoint with the maximum $p_k$ was excluded; and this step was repeated until all $p$ values were smaller than the termination p value ($p_{final}$) for the chromosome;

③ acquisition of the termination $p$ value: in the test process, the above-mentioned steps a) to c) ① were performed by using the control sample as the test sample, for the chromosome c, all sorted candidate breakpoints of the cth chromosome were recorded as $B_c = \{b_1, b_2,...,b_s\}$ , wherein two windows, one on the left and the other on the right, exist for each candidate breakpoint $b_k$; the $p$ value ($p_k$) obtained by subjecting all $Z_{i,j}$ in these two windows to the run test was regarded as "the significance of $b_k$ as a breakpoint"; a candidate breakpoint of the least significance was excluded, until the number of candidate breakpoints was equal to the expected value $N_c$ ($N_c = L_c/T$ , where $L_c$ is the length of the chromosome, and the theoretical ultimate precision $T$ = 2 Mb); and in the set of the candidate breakpoints, the minimum $p$ value was the termination $p$ value ($p_{final}$) of the chromosome, see the following table;

Relevant values used in the example

| Chromosome | Length of Chromosome (bp) | $N_c$ | $p_{final}$ |
|---|---|---|---|
| 1 | 247,249,719 | 123 | 4.45E-131 |
| 2 | 242,951,149 | 121 | 2.30E-169 |
| 3 | 199,501,827 | 99 | 4.98E-149 |
| 4 | 191,273,063 | 95 | 5.11E-172 |
| 5 | 180,857,866 | 90 | 4.47E-141 |
| 6 | 170,899,992 | 85 | 1.99E-127 |
| 7 | 158,821,424 | 79 | 2.70E-145 |
| 8 | 146,274,826 | 73 | 2.31E-131 |
| 9 | 140,273,252 | 70 | 2.99E-121 |
| 10 | 135,374,737 | 67 | 1.22E-206 |
| 11 | 134,452,384 | 67 | 2.99E-121 |
| 12 | 132,349,534 | 66 | 2.60E-127 |
| 13 | 114,142,980 | 57 | 2.10E-178 |
| 14 | 106,368,585 | 53 | 2.51E-67 |
| 15 | 100,338,915 | 50 | 2.77E-128 |
| 16 | 88,827,254 | 44 | 2.70E-84 |
| 17 | 78,774,742 | 39 | 1.27E-89 |
| 18 | 76,117,153 | 38 | 1.07E-143 |
| 19 | 63,811,651 | 31 | 1.71E-120 |
| 20 | 62,435,964 | 31 | 1.56E-95 |
| 21 | 46,944,323 | 23 | 3.96E-89 |
| 22 | 49,691,432 | 24 | 2.38E-111 |

d) fragment filtration after window merging: in order to further filter fragments obtained after window merging, the average of $Z_{i,j}$ in the fragment was calculated and recoded as $\overline{Z}$ , and if $\overline{Z}$ of the fragment was smaller than -1.28 or greater than 1.28, then the fragment was a copy number variation. See Table 1 for the results.

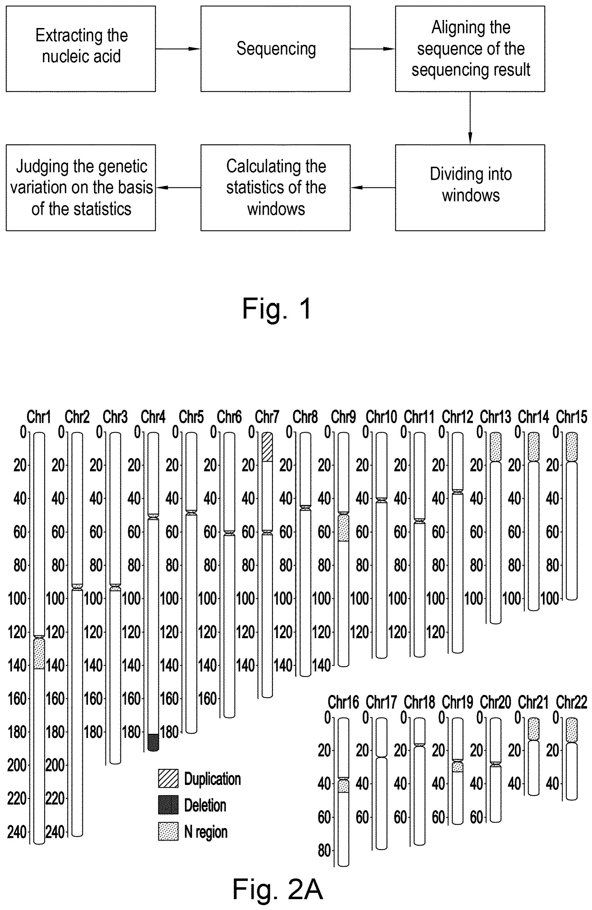

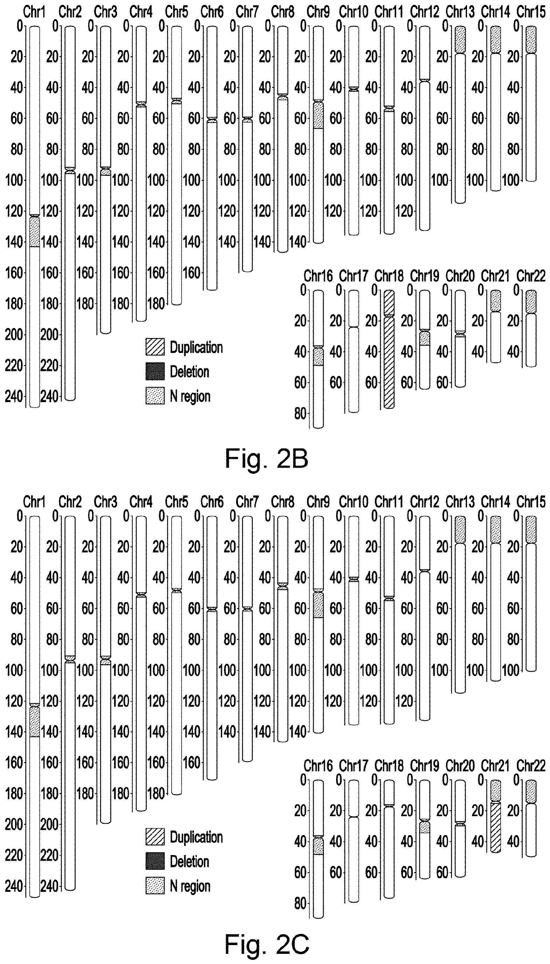

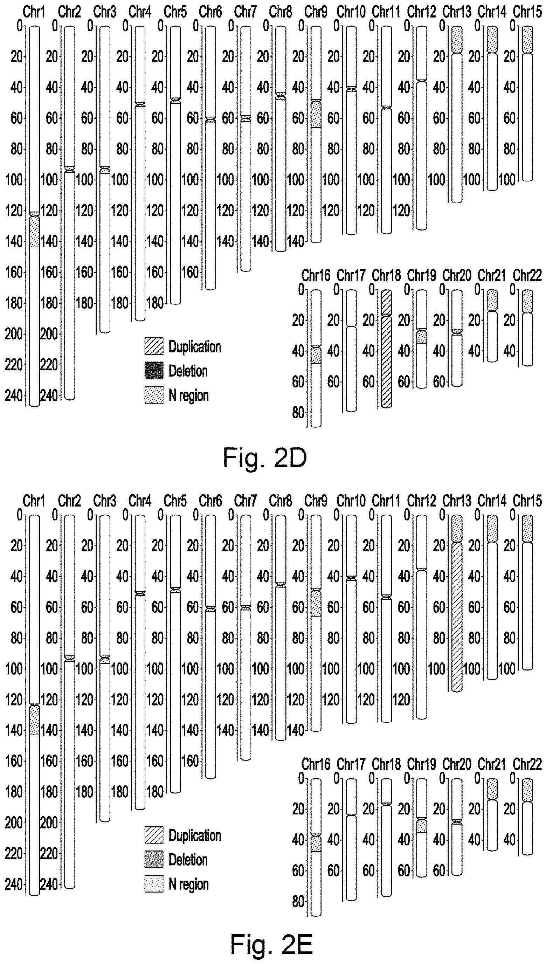

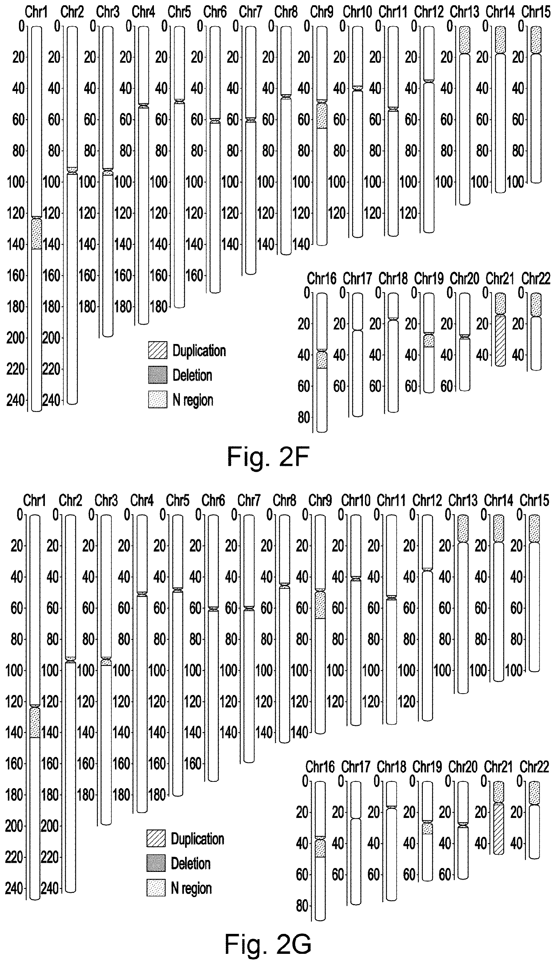

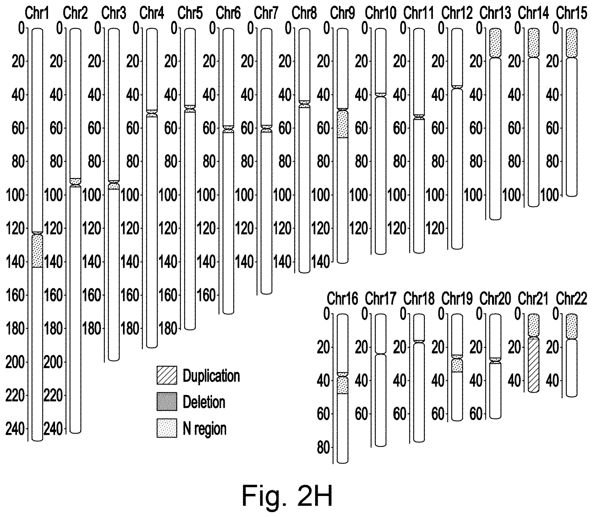

4) Result visualization, see Figure 2.

Table 1. A list of CNV results of all samples in the implementation case

| No. | Chromo some | CNV starting point | CNV ending point | CNV size | Judgment result | Regions and bands involved |
|---|---|---|---|---|---|---|
| S67 | 4 | 181,243,323 | 191,250,465 | 10.1M | Deletion | 4q34.3→q35.2 |
| | 7 | 34,983 | 17,074,358 | 17M | Duplication | 7p21.1→p22.3 |
| S10 | 18 | 1 | 76,117,153 | 76.1M | Duplication | 18p11.32→q23 |
| S14 | 21 | 1 | 46,944,323 | 46.9M | Duplication | 21p13→q22.3 |
| S18 | 18 | 1 | 76,117,153 | 76.1M | Duplication | 18p11.32→q23 |
| S49 | 13 | 1 | 114,142,980 | 114.1M | Duplication | 13p13→q34 |
| S55 | 21 | 1 | 46,944,323 | 46.9M | Duplication | 21p13→q22.3 |
| S82 | 21 | 1 | 46,944,323 | 46.9M | Duplication | 21p13→q22.3 |
| S103 | 13 | 1 | 114,142,980 | 114.1M | Duplication | 13p13→q34 |

The results of CNV analysis in the present invention were compared with the results by the CGH chip in the following, and the comparison results are shown as the following Table 2. For the results by the CGH chip, the Human Genome CGH Microarray Kit (Agilent Technologies Inc.) was used.

Same was obtained according to the protocol by the provider, and the steps are briefly described as follows: DNA from a healthy person with the same sex as the sample to be tested or mixed DNA from male and female healthy persons was used as the reference DNA, the reference DNA and the DNA to be tested were labeled with the fluoresceins, Cy3 and Cy5, respectively, and then hybridized with probes, and if the fluorescence intensity ratio of the DNA to be tested to the reference DNA was 1, then it could be understood as that the amounts of the DNA to be tested and that of the reference DNA were equal, and if the ratio was not equal to 1, then it was indicated that there is deletions or amplifications in the DNA to be tested. The resolutions of various types of Array CGH depend on the interval between and the length of the probes on the microarray. Flow: remaining cell culture liquid after the G-banding chromosome examination was collected, and genomic DNAs of the sample to be tested and that of the control sample were extracted. After purification, the sample to be tested and the reference sample were fluorescently labeled differently, and then the samples were mixed with Cot-1 DNA blocking non-specific hybridization, denatured, pre-annealed, and hybridized with the microarray, and finally, DNA that did not bind to the microarray in a target specific manner was eluted, and the fluorescence intensity ratio of the two signals on the target on each microarray was obtained after scanning and analysis by software, which reflected the change in the copy number of a corresponding sequence or gene between the genomic DNA of the sample to be tested and the genomic DNA of the reference sample.

Table 2. Comparison of the detection results of the example of the present invention and the results by the CGH chip

| No. | Chromosome | Results by the CGH chip | Detection results of the method of the present invention | Judgment result |
|---|---|---|---|---|
| S67 | 4 | Deletion: 181,170,528-190,958,960 | Deletion: 181,243,323-191,250,465 | Consistent |
| | 7 | Duplication: 204,709-16,283,844 | Duplication: 34,983-17,074,358 | Consistent |

The results of CNV analysis in the present invention were compared with the results of standard karyotyping in the following, and the comparison results were shown as the following Table 3.The steps of standard karyotyping were as follows:

(1) The amniotic fluid obtained by centesis was centrifuged for 5 minutes (at a rotational speed of 800-1,000 revolution/minute), and then inoculated in an inoculation hood. The supernatant was pipetted out and retained for other examinations, 0.5 ml of amniotic fluid and precipitated amniotic fluid cells remained in the centrifuge tube, and the precipitated fetal exfoliated cells and amniotic cells were pipetted uniformly into a cell suspension, and inoculated into three culture flasks containing a culture solution.

(2) The culture flasks were placed in a carbon dioxide incubator.

(3) 5-7 days after inoculation, viable cells in the amniotic fluid were adhered to the bottoms of the flasks, and began to grow, and the growth status of the cells could be observed with an inverted microscope. If adherence had occurred, the culture solution could be changed, 3-5 ml of the fresh culture solution was added, and the solution was changed once every 2-3 days afterwards. Adherent cells included epithelioid cells, fibroblast-like cells and amniotic fluid cells which were a kind of cells with the morphology falling between the epithelioid cells and fibroblasts. The above-mentioned three kinds of cells all formed clones, and if the growth status was good, 11-14 days after inoculation, there could be more than ten large-flake clones at the bottoms of the flasks, unaided eyes could also see flaky clones at the bottoms of the flasks, and the cell nuclei were large and round. At this time, smear preparation, or referred to as harvest, could be prepared. One day before harvest, same should be changed with the fresh culture solution to increase nucleus division.

(4) Harvest: harvest was performed 14-20 days after culture on average. Colchicine of 0.04 nanogram/milliliter was added into the culture flasks, the cells were made to be halted at the metaphase and cultured for 5-15 hours, and it could be seen under an inverted microscope that there were many cell nuclei at the mitotic phase, and the cells were round and large and bright as a flake of bright pearls, and were interlinked. The amount of colchicine added could be different in various laboratories.

(5) Trypsinizing: the culture solution in the culture flasks was poured into centrifuge tubes, 0.5 ml of 0.02% EDTA trypsin digestion solution or 0.5 ml of 0.15% pronase was placed at the bottoms of the culture flasks, the cell clones at the bottoms of the flasks were pipetted gently with a long curved glass pipette, it was seen under an inverted microscope that clone cells had floated, same was pipetted into the centrifuge tubes, and then, cells that had not yet floated were washed with 0.5-1 ml of Hank's solution, continued to be pipetted with the long pipette and were poured into the centrifuge tubes after being made to be completely detached. Centrifugation was performed for 5 minutes at a speed of 800-1,000 revolution/minute, the supernatant was removed and the cells were reserved for use.

(6) Hypotonic treatment: 4 ml of 0.075M KCl solution at 37°C was added gently into the above-mentioned centrifuge tubes and cells, the bottoms of the tubes were flicked gently with a finger or the precipitated cells were dispersed with a sharp pipette, same were placed in a 37°C water bath for 16 minutes (the time of hypotonic treatment could be determined according to their own experiences in various laboratories), and centrifuged for 5 minutes, the supernatant was removed, the fresh fixation solution (methanol: glacial acetic acid = 3 : 1) was dropped gently in along the walls of the tubes, the bottoms of the tubes were patted gently with a finger, the cells were separated uniformly, fixed for 15 minutes and centrifuged, the fixation solution was changed, and after second-time fixation was performed for 30 minutes, same overnighted.

(7) Smear blowing: after centrifugation, the supernatant was removed and 0.5 ml was retained and prepared into a cell suspension, or the supernatant was completely removed and 0.5 ml of newly-prepared fixation solution was added, and after same is pipetted carefully with a long and thin glass tube, one drop was pipetted out, dropped onto a glass slide taken out from ice water, and dispersed by blowing gently, and after the glass slide was placed in the air and dried, the dispersion status of chromosomes was observed under a microscope, and then smear blowing was continued. The dried glass slide could be directly stained with Giemsa.

(8) Banding: if the chromosomal morphology was good, Giemsa banding, referred to as G-banding, could be performed. The glass slide was firstly baked at 65°C for 1 hour, or baked at 37°C for 24 hours, the glass slide was placed in 0.25% trypsin solution for 20-25 seconds at room temperature, subjected to physiological saline twice, placed in 2% Giemsa solution for 5-10 minutes, taken out, washed with running water, and dried in the air, and the chromosomes could be observed under a microscope to perform karyotyping.

Table 3. Comparison of the detection results in the present implementation case and the standard karyotyping detection results

| No. | Standard karyotyping | Detection results of the method of the present invention | Judgment result |
|---|---|---|---|
| S10 | T18 | T18 | Consistent |

(continued)

| No. | Standard karyotyping | Detection results of the method of the present invention | Judgment result |
|---|---|---|---|
| S14 | T21 | T21 | Consistent |
| S18 | T18 | T18 | Consistent |
| S49 | T13 | T13 | Consistent |
| S55 | T21 | T21 | Consistent |
| S82 | T21 | T21 | Consistent |
| S103 | T13 | T13 | Consistent |

Although the particular embodiments of the present invention have been detailed, it will be understood by those skilled in the art that according to all teachings that have been disclosed that those details can be subjected to various modifications and substitutions.

**Claims**

1. A computer-implemented method of detecting genetic variation, comprising the following steps:

   1) acquiring reads from a test sample;
   2) aligning said reads with a reference genome sequence;
   3) dividing said reference genome sequence into windows, calculating the number of reads which are aligned to each window, and acquiring the statistic for each window on the basis of the number of said reads;
   4) for a fragment of the reference genome sequence, on the basis of the change in the statistics of all the windows thereon in the fragment of the reference genome sequence, acquiring positions where a significant change occurs in statistics of the windows on both sides, these positions being positions where genetic variation sites of the test sample are on the reference genome sequence; and
   5) screening the genetic variation sites to obtain post-screening genetic variation sites, wherein for each genetic variation site, performing statistical tests on the difference between two numerical groups consisting of statistics of windows contained in the fragment between the genetic variation site and its preceding genetic variation site and in the fragment between the genetic variation site and its subsequent genetic variation site, and removing the genetic variation site whose significance value of difference is maximum and greater than a preset threshold; and repeating the above-mentioned process, until the significance values of difference of the genetic variation sites are all smaller than the preset threshold; and
   wherein the preset threshold is acquired by the following steps:

   a) acquiring the genetic variation sites according to steps 1) to 4) by substituting the test sample with a control sample,
   b) for each genetic variation site, performing statistics on the difference between two numerical groups consisting of statistics of windows contained in the fragment between the genetic variation site and its preceding genetic variation site and in the fragment between the genetic variation site and its subsequent variation site, and removing the genetic variation site which is the least significant; and
   c) repeating the above-mentioned step b), until a number of remaining candidate breakpoints is equal to the expected value $N_c$, wherein $N_c = L_c/T$, $L_c$ is the length of the genome sequence, the theoretical ultimate precision $T$ is the fragment size which can be detected theoretically, the theoretical ultimate precision $T = W+S*N$ when the average of the window sizes is W, the sliding length of the windows is S and the number of each window group in the run test is N, and among significance values of all the remaining candidate breakpoints, the minimum is the significance threshold.

2. A computer-implemented method of detecting genetic variation as claimed in claim 1, wherein the length of said reads is 25-100 nt.

3. A computer-implemented method of detecting genetic variation as claimed in claim 1, wherein the length of said reads is 35-100 nt.

4. A computer-implemented method of detecting genetic variation as claimed claim 1, wherein the number of said reads is at least 1 million.

5. A computer-implemented method of detecting genetic variation as claimed in claim 1, wherein said windows have the same number of reference unique reads.

6. A computer-implemented method of detecting genetic variation as claimed in claim 1, wherein said windows have an overlap or have no overlap therebetween.

7. A computer-implemented method of detecting variation as claimed in claim 1, wherein said statistic approximately fits normal distribution obtained by a standardization processing on the number of reads which are aligned to a window.

8. A computer-implemented method of detecting genetic variation as claimed in claim 7, wherein said standardization is based on the average number of reads which are aligned to all the windows.

9. A computer-implemented method of detecting genetic variation as claimed in claim 1, wherein said genetic variation site is the median point between an inflection point where said statistic turns from ascending to descending and the next same inflection point, and there is at least 50, at least 70, at least 100, preferably 100 window lengths between two genetic variation sites.

10. A computer-implemented method of detecting genetic variation as claimed in claim 1, wherein in step 5) said significance value of difference is performed by the run test, removing the genetic variation site whose significance value in the run test is maximum and greater than the preset threshold; and repeating the above-mentioned process, until the significance values of the genetic variation sites in the run test are all smaller than the preset threshold.

11. A computer-implemented method of detecting genetic variation as claimed in any preceding claim, comprising the following steps:

   1) acquiring the genetic variation sites on a fragment of the reference genome sequence according to the method of any preceding claim; and
   2) performing a confidence-based selection on fragments between said genetic variation sites.

12. A computer-implemented method of detecting genetic variation as claimed in claim 11, wherein said step 2) is:

   i) calculating the distribution probability of the statistics through the distribution pattern of the statistics for the windows, and setting a threshold; and
   ii) comparing the average of the statistics of windows in the fragment between post-screening genetic variation sites with said threshold, and determining whether the fragment between the genetic sites is anomalous on the basis of the comparison result.

13. A computer-implemented method of detecting genetic variation as claimed in claim 11, wherein said step 2) is:

   i) calculating the distribution probability of the statistics through the distribution pattern of the statistics for the windows, and setting a first threshold and a second threshold; and
   ii) comparing the average of the statistics of windows in the fragment between post-screening genetic variation sites with said first threshold and second threshold,

   wherein, if the statistics for windows in the fragment are smaller than the first threshold, the fragment is a fragment deletion, and if same are greater than the second threshold, the fragment is a fragment duplication.

14. A computer-implemented method of detecting genetic variation as claimed in claim 13, wherein said first threshold is a value of the statistic where the cumulative probability is 0.05, and/or said second threshold is a value of the statistic where the cumulative probability is 0.95.

15. A computer-implemented method of detecting genetic variation as claimed in any preceding claim, wherein the test sample is a maternal sample containing fetal nucleic acid; preferably wherein the maternal sample is maternal peripheral blood.

**Patentansprüche**

1. Computerimplementiertes Verfahren zum Nachweis genetischer Variation, wobei das Verfahren die folgenden Schritte umfasst:

   1) Erfassen von Lesewerten einer Testprobe;

   2) Abstimmen der Lesewerte mit einer Referenz-Genomsequenz;

   2) Unterteilen der Referenz-Genomsequenz in Fenster, wobei die Anzahl der Lesewerte berechnet wird, die mit jedem Fenster abgestimmt werden, und wobei die Statistik für jedes Fenster auf der Basis der Anzahl der Lesewerte erfasst wird;

   4) für ein Fragment der Referenz-Genomsequenz, auf der Basis der Veränderung in der Statistik aller Fenster daran in dem Fragment der Referenz-Genomsequenz Erfassen von Positionen, an denen eine signifikante Veränderung auftritt in der Statistik der Fenster auf beiden Seiten, wobei diese Positionen Positionen sind, an denen genetische Variationsstellen der Testprobe sich auf der Referenz-Genomsequenz befinden; und

   5) Screening der genetischen Variationsstellen, um Post-Screening genetische Variationsstellen zu erhalten, wobei für jede genetische Variationsstelle statistische Tests an der Differenz zwischen zwei numerischen Gruppen durchgeführt werden, die aus Statistiken von Fenstern bestehen, die in dem Fragment zwischen der genetischen Variationsstelle und der vorangehenden genetischen Variationsstelle enthalten sind, und in dem Fragment zwischen der genetischen Variationsstelle und der folgenden genetischen Variationsstelle, und wobei die genetische Variationsstelle entfernt wird, deren Signifikanzwert der Differenz maximal ist und größer als ein voreingestellter Grenzwert; und wobei der vorstehend genannte Prozess wiederholt wird, bis die Signifikanzwerte der Differenz der genetischen Variationsstellen alle kleiner sind als der voreingestellte Grenzwert; und

   wobei der voreingestellte Grenzwert durch die folgenden Schritte ermittelt wird:

   a) Erfassen der genetischen Variationsstellen gemäß den Schritten 1) bis 4) durch Substituieren der Testprobe durch eine Kontrollprobe;

   b) für jede genetische Variationsstelle, Durchführen von Statistiken an der Differenz zwischen zwei numerischen Gruppen, welche aus Statistiken von Fenstern bestehen, die in dem Fragment zwischen der genetischen Variationsstelle und der vorangehenden genetischen Variationsstelle enthalten sind, und in dem Fragment zwischen der genetischen Variationsstelle und der folgenden genetischen Variationsstelle, und Entfernen der genetischen Variationsstelle, die am wenigsten signifikant ist; und

   c) Wiederholen des oben genannten Schritts b), bis eine Anzahl verbliebener Bruchstellen gleich dem erwarteten Wert $N_c$ ist, wobei $N_c = L_c/T$, wobei $L_c$ die Länge der Genomsequenz ist, wobei die theoretische ultimative Präzision T die Fragmentgröße ist, die theoretisch nachgewiesen werden kann, wobei die theoretische ultimative Präzision $T = W + S * N$ entspricht, wobei der Mittelwert der Fenstergrößen W ist, wobei die Schiebelänge des Fensters S ist, und wobei die Anzahl jeder Fenstergruppe in dem durchgeführten Test N ist, und wobei unter allen Signifikanzwerten aller verbliebenen möglichen Bruchstellen das Minimum der Signifikanzgrenzwert ist.

2. Computerimplementiertes Verfahren zum Nachweis genetischer Variation nach Anspruch 1, wobei die Länge der Lesewerte 25 - 100 nt ist.

3. Computerimplementiertes Verfahren zum Nachweis genetischer Variation nach Anspruch 1, wobei die Länge der Lesewerte 35 - 100 nt ist.

4. Computerimplementiertes Verfahren zum Nachweis genetischer Variation nach Anspruch 1, wobei die Anzahl der Lesewerte mindestens 1 Million beträgt.

5. Computerimplementiertes Verfahren zum Nachweis genetischer Variation nach Anspruch 1, wobei die Fenster die gleiche Anzahl an eindeutigen Referenz-Lesewerten aufweisen.

6. Computerimplementiertes Verfahren zum Nachweis genetischer Variation nach Anspruch 1, wobei die Fenster dazwischen eine Überlappung oder keine Überlappung aufweisen.

7. Computerimplementiertes Verfahren zum Nachweis genetischer Variation nach Anspruch 1, wobei die Statistik ungefähr mit einer Normalverteilung übereinstimmt, die durch eine Standardisierungsverarbeitung an der Anzahl an Lesewerten erhalten wird, die mit einem Fenster abgestimmt sind.

**8.** Computerimplementiertes Verfahren zum Nachweis genetischer Variation nach Anspruch 7, wobei die Standardisierung auf der durchschnittlichen Anzahl von Lesewerten basiert, die mit allen Fenstern abgestimmt sind.

**9.** Computerimplementiertes Verfahren zum Nachweis genetischer Variation nach Anspruch 1, wobei die genetische Variationsstelle der Mittelpunkt zwischen einem Wendepunkt ist, wo die Statistik von aufsteigend in absteigend wechselt, und dem nächsten gleichen Wendepunkt, und wobei zwischen zwei genetischen Variationsstellen mindestens 50, mindestens 70, mindestens 100, vorzugsweise 100 Fensterlängen liegen.

**10.** Computerimplementiertes Verfahren zum Nachweis genetischer Variation nach Anspruch 1, wobei in Schritt 5) der Signifikanzwert der Differenz durch den ausgeführten Test ausgeführt wird, wobei die genetische Variationsstelle entfernt wird, deren Signifikanzwert in dem ausgeführten Test maximal ist und größer ist als der voreingestellte Grenzwert; und wobei der vorstehend genannte Prozess wiederholt wird, bis die Signifikanzwerte der genetischen Variationsstellen in dem ausgeführten Test alle kleiner sind als der voreingestellte Grenzwert.

**11.** Computerimplementiertes Verfahren zum Nachweis genetischer Variation nach einem der vorstehenden Ansprüche, wobei dieses die folgenden Schritte umfasst:

1) Erfassen der genetischen Variationsstellen an einem Fragment der Referenz-Genomsequenz gemäß dem Verfahren nach einem der vorstehenden Ansprüche; und

2) Durchführen einer auf Sicherheit basierenden Auswahl an Fragmenten zwischen den genetischen Variationsstellen.

**12.** Computerimplementiertes Verfahren zum Nachweis genetischer Variation nach Anspruch 11, wobei Schritt 2) wie folgt ist:

i) Berechnen der Verteilungswahrscheinlichkeit der Statistik über das Verteilungsmuster der Statistik für die Fenster, und Festlegen eines Grenzwerts; und

ii) Vergleichen des Durchschnitts der Statistik der Fenster in dem Fragment zwischen Post-Screening genetischen Variationsstellen mit dem Grenzwert, und auf der Basis des Vergleichsergebnisses Bestimmen, ob das Fragment zwischen den genetischen Stellen anomal ist.

**13.** Computerimplementiertes Verfahren zum Nachweis genetischer Variation nach Anspruch 11, wobei Schritt 2) wie folgt ist:

i) Berechnen der Verteilungswahrscheinlichkeit der Statistik über das Verteilungsmuster der Statistik für die Fenster, und Festlegen eines ersten Grenzwerts und eines zweiten Grenzwerts; und

ii) Vergleichen des Durchschnitts der Statistik der Fenster in dem Fragment zwischen Post-Screening genetischen Variationsstellen mit dem ersten Grenzwert und dem zweiten Grenzwert;

wobei, wenn die Statistik für Fenster in dem Fragment kleiner ist als der erste Grenzwert, das Fragment eine Fragmentlöschung ist, und wenn die Statistik größer ist als der zweite Grenzwert, das Fragment eine Fragmentduplikation ist.

**14.** Computerimplementiertes Verfahren zum Nachweis genetischer Variation nach Anspruch 13, wobei der erste Grenzwert ein Statistikwert ist, wobei die kumulative Wahrscheinlichkeit 0,05 ist, und/oder wobei der zweite Grenzwert ein Statistikwert ist, wobei die kumulative Wahrscheinlichkeit 0,95 ist.

**15.** Computerimplementiertes Verfahren zum Nachweis genetischer Variation nach einem der vorstehenden Ansprüche, wobei die Testprobe eine Mutterprobe ist, die fetale Nucleinsäure enthält; wobei die Mutterprobe vorzugsweise mütterliches peripheres Blut ist.

**Revendications**

**1.** Procédé mis en oeuvre par ordinateur pour détecter une variation génétique, comprenant les étapes consistant à :

1) acquérir des lectures à partir d'un échantillon à tester ;
2) aligner lesdites lectures avec une séquence du génome de référence ;

3) diviser ladite séquence du génome de référence en fenêtres, calculer le nombre de lectures qui sont alignées avec chaque fenêtre, et acquérir la statistique pour chaque fenêtre sur la base du nombre desdites lectures ;

4) pour un fragment de la séquence du génome de référence, sur la base du changement dans les statistiques de toutes les fenêtres du fragment de la séquence du génome de référence, acquérir des positions où un changement significatif se produit dans les statistiques des fenêtres des deux côtés, ces positions étant des positions où les sites de variation génétique de l'échantillon à tester sont sur la séquence du génome de référence, et

5) cribler les sites de variation génétique pour obtenir des sites de variation génétique post-criblage, pour chaque site de variation génétique, réaliser des tests statistiques sur la différence entre deux groupes numériques consistant en des statistiques de fenêtres contenues dans le fragment entre le site de variation génétique et son site de variation génétique précédent et dans le fragment entre le site de variation génétique et son site de variation génétique suivant, et retirer le site de variation génétique dont la valeur significative de différence est maximale et supérieure à un seuil prédéfini ; et répéter le processus susmentionné, jusqu'à ce que les valeurs significatives de différence des sites de variation génétique soient inférieures au seuil prédéfini ; et

le seuil prédéfini étant acquis par les étapes suivantes consistant à :

a) acquérir les sites de variation génétique selon les étapes 1) à 4) en substituant l'échantillon à tester par un échantillon témoin,

b) pour chaque site de variation génétique, effectuer des statistiques sur la différence entre deux groupes numériques consistant en des statistiques de fenêtres contenues dans le fragment entre le site de variation génétique et son site de variation génétique précédent et dans le fragment entre le site de variation génétique et son site de variation ultérieur, et supprimer le site de variation génétique qui est le moins significatif ; et

c) répéter l'étape b) susmentionnée, jusqu'à ce qu'un nombre de points de rupture candidats restants soit égal à la valeur attendue $Nc$, $N_c = Lc/T$, $L_c$ étant la longueur de la séquence du génome, la précision théorique ultime T étant la taille du fragment qui peut être détecté théoriquement, la précision théorique ultime $T = W+S*N$ lorsque la moyenne des tailles des fenêtres est W, la longueur de glissement des fenêtres est S et le nombre de chaque groupe de fenêtres dans le test effectué étant N, et entre valeurs significatives des points de rupture candidats restant, le minimum étant le seuil significatif.

2. Procédé mis en oeuvre par ordinateur pour détecter une variation génétique selon la revendication 1, la longueur desdites lectures étant 25-100 nt.

3. Procédé mis en oeuvre par ordinateur pour détecter une variation génétique selon la revendication 1, la longueur desdites lectures étant 35-100 nt.

4. Procédé mis en oeuvre par ordinateur pour détecter une variation génétique selon la revendication 1, le nombre desdites lectures étant d'au moins 1 million.

5. Procédé mis en oeuvre par ordinateur pour détecter une variation génétique selon la revendication 1, lesdites fenêtres ayant le même nombre de lectures uniques de référence.

6. Procédé mis en oeuvre par ordinateur pour détecter une variation génétique selon la revendication 1, lesdites fenêtres ayant un chevauchement ou n'ayant pas de chevauchement entre elles.

7. Procédé mis en oeuvre par ordinateur pour détecter une variation selon la revendication 1, ladite statistique correspondant approximativement à une distribution normale obtenue par un traitement de normalisation sur le nombre de lectures qui sont alignées avec une fenêtre.

8. Procédé mis en oeuvre par ordinateur pour détecter une variation génétique selon la revendication 7, ladite normalisation étant basée sur le nombre moyen de lectures qui sont alignées avec toutes les fenêtres.

9. Procédé mis en oeuvre par ordinateur pour détecter une variation génétique selon la revendication 1, ledit site de variation génétique étant le point médian entre un point d'inflexion où ladite statistique passe du point ascendant au point descendant et le point d'inflexion suivant, et au moins 50, au moins 70, au moins 100, de préférence 100 fenêtres se trouvant entre deux sites de variation génétique.

10. Procédé mis en oeuvre par ordinateur pour détecter une variation génétique selon la revendication 1, à l'étape 5), ladite valeur significative de différence étant réalisée par le test effectué, comprenant les étapes consistant à retirer le site de variation génétique dont la valeur significative dans le test effectué est maximale et supérieure au seuil prédéfini ; et répéter le processus susmentionné, jusqu'à ce que les valeurs significatives des sites de variation génétique dans le test effectué soient toutes inférieures au seuil prédéfini.

11. Procédé mis en oeuvre par ordinateur pour détecter une variation génétique selon l'une quelconque des revendications précédentes, comprenant les étapes suivantes consistant à :

   1) acquérir les sites de variation génétique sur un fragment de la séquence du génome de référence selon le procédé selon l'une quelconque des revendications précédentes ; et
   2) effectuer une sélection basée sur la confiance sur des fragments entre lesdits sites de variation génétique.

12. Procédé mis en oeuvre par ordinateur pour détecter une variation génétique selon la revendication 11, ladite étape 2) consistant à :

   i) calculer la probabilité de distribution des statistiques au moyen du modèle de distribution des statistiques pour les fenêtres et établir un seuil ; et
   ii) comparer la moyenne des statistiques des fenêtres dans le fragment entre les sites de variation génétique post-dépistage et ledit seuil, et déterminer si le fragment entre les sites génétiques est anormal sur la base du résultat de la comparaison.

13. Procédé mis en oeuvre par ordinateur pour détecter une variation génétique selon la revendication 11, ladite étape 2) consistant à :

   i) calculer la probabilité de distribution des statistiques au moyen du modèle de distribution des statistiques pour les fenêtres, et établir un premier seuil et un second seuil ; et
   ii) comparer la moyenne des statistiques des fenêtres dans le fragment entre les sites de variation génétique post-screening avec lesdits premier et second seuils,

si les statistiques pour les fenêtres du fragment sont inférieures au premier seuil, le fragment étant une délétion de fragment, et si elles sont supérieures au second seuil, le fragment étant une duplication de fragment.

14. Procédé mis en oeuvre par ordinateur pour détecter une variation génétique selon la revendication 13, ledit premier seuil étant une valeur de la statistique où la probabilité cumulative est 0,05, et/ou ledit second seuil étant une valeur de la statistique où la probabilité cumulative est 0,95.

15. Procédé mis en oeuvre par ordinateur pour détecter une variation génétique selon l'une quelconque des revendications précédentes, l'échantillon à tester étant un échantillon maternel contenant un acide nucléique foetal ; de préférence l'échantillon maternel étant du sang périphérique maternel.

Fig. 1

Fig. 2A

Fig. 2B

Fig. 2C

Fig. 2D

Fig. 2E

Fig. 2F

Fig. 2G

Fig. 2H

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Down's syndrome and fragile X syndrome. Genetics of Mental Retardation. Monogr Hum Genet. Basel, Karger. 2010, vol. 18, 101-113
- **BRETELLE F et al.** Prenatal and postnatal diagnosis of 22q11.2 deletion syndrome. *Eur J Med Genet.,* November 2010, vol. 53 (6), 367-70
- **MALCOLM S.** Microdeletion and microduplication syndromes. *Prenat Diagn.,* December 1996, vol. 16 (13), 1213-9
- **DAVID PETERS et al.** Noninvasive Prenatal Diagnosis of a Fetal Microdeletion Syndrome. *N Engl J Med,* 2011, vol. 365, 1847-1848
- **RUSK ; NICOLE.** Cheap Third-Generation Sequencing. *Nature Methods,* 01 April 2009, vol. 6 (4), 2446
- **METZKER ML.** Sequencing technologies-the next generation. *Nat Rev Genet,* January 2010, vol. 11 (1), 31-46
- **MICAH HAMADY ; JEFFREY J WALKER ; J KIRK HARRIS et al.** Error-correcting barcoded primers forpyrosequencing hundreds of samples in multiplex. *Nature Methods,* March 2008, vol. 5 (3